# EUROPÄISCHE PATENTANMELDUNG

(11) **EP 4 258 273 A1**
(43) Veröffentlichungstag der Anmeldung: **11.10.2023**
(21) Anmeldenummer: 23164262.0
(22) Anmeldetag: 27.03.2023
(51) Int. Cl.: G16H 10/60, G16H 40/60

(54) **MEDIZINISCHES SYSTEM UND VERFAHREN**

(30) Priorität: 04.04.2022 DE 102022107947
(71) Anmelder: Drägerwerk AG & Co. KGaA, 23558 Lübeck (DE)
(72) Erfinder: Krüger, Thomas, 23558 Lübeck (DE); Landwehr, Birger, 23558 Lübeck (DE); Franz, Frank, 23558 Lübeck (DE)

(57) **Zusammenfassung**

Die Erfindung betrifft ein medizinisches System (100) zur Bereitstellung einer Einstellungsempfehlung (105) für ein Beatmungsgerät (102) für die Beatmung eines Patienten, mit einer Datenschnittstelle (110), einer Messwertschnittstelle (120), einer Regelspeichereinheit (130) und einer Verarbeitungseinheit (150). Die Datenschnittstelle ist ausgebildet, statische Patientendaten (112) zu empfangen, wobei statische Patientendaten Werte von Patientenparametern umfassen, die im Laufe einer Behandlungsdauer im Wesentlichen unveränderlich sind. Die Messwertschnittstelle ist ausgebildet, dynamische Patientendaten (122) zu empfangen, wobei dynamische Patientendaten Werte von Patientenparametern umfassen, die sich im Laufe der Behandlungsdauer verändern. Die Verarbeitungseinheit ist ausgebildet, unmittelbar nach dem Empfang der Patientendaten einen neuen aktuellen Patientendatensatz (152) basierend auf den aktuell verfügbaren Patientendaten zu bestimmen. Zudem ist die Verarbeitungseinheit ausgebildet, ausgelöst durch das Bestimmen des neuen aktuellen Patientendatensatzes einen Vergleich zwischen den Werten des aktuellen Patientendatensatzes und hinterlegten Zuordnungsregeln (132) zu initiieren und basierend auf diesem Vergleich mindestens eine angesichts des aktuellen Patientendatensatzes empfohlene Geräteeinstellung (134) des Beatmungsgerätes von der Regelspeichereinheit zu empfangen, und basierend auf einem Vergleich mit einer aktuellen Geräteeinstellung (154) eine Empfehlungsausgabe (156) auszulösen.

## Beschreibung

Die Erfindung betrifft ein medizinisches System zur Bereitstellung einer Einstellungsempfehlung für ein Beatmungsgerät für die Beatmung eines Patienten. Weiterhin betrifft die Erfindung ein Verfahren zur Bereitstellung einer Einstellungsempfehlung für ein Beatmungsgerät für die Beatmung eines Patienten.

Grundsätzlich ist die Bestimmung von Empfehlungen für den Betrieb von Beatmungsgeräten bekannt. So ist beispielsweise eine automatische Erkennung von Körper-Metriken, wie etwa Größe und Gewicht für eine darauffolgende Anpassung von Alarmeinstellungen bekannt. Ebenfalls bekannt ist die Verwendung von sogenannten Expertensystemen, bei denen basierend auf physiologischen Modellen eine Behandlungsempfehlung ausgegeben und/oder eine entsprechende Behandlung automatisiert ausgeführt wird.

Unter einem Beatmungsgerät ist im Folgenden ein Gerät zu verstehen, welches einen Patienten beatmet. Also beispielsweise sind neben Geräten, die ausschließlich zur Beatmung verwendet werden, auch Anästhesiegeräte Beatmungsgeräte im Sinne dieser Erfindung.

Aufgabe der vorliegenden Erfindung ist es, ein verbessertes medizinisches System, insbesondere ein System, welches nachvollziehbar und schnell zu Behandlungsempfehlungen gelangt, bereitzustellen.

Erfindungsgemäß wird zur Lösung dieser Aufgabe ein medizinisches System zur Bereitstellung einer Einstellungsempfehlung für ein Beatmungsgerät für die Beatmung eines Patienten vorgeschlagen, mit einer Datenschnittstelle, einer Messwertschnittstelle, einer Regelspeichereinheit, einer Verarbeitungseinheit und einer Ausgabeeinheit.

Die Datenschnittstelle ist ausgebildet, statische Patientendaten zumindest aus einer Patientendatenbank zu empfangen, wobei statische Patientendaten Werte von Patientenparametern umfassen, die im Laufe einer Behandlungsdauer im Wesentlichen unveränderlich sind.

Die Messwertschnittstelle ist ausgebildet, dynamische Patientendaten zumindest von einem Beatmungsgerät zu empfangen, wobei dynamische Patientendaten Werte von Patientenparametern umfassen, die sich im Laufe der Behandlungsdauer verändern.

In der Regelspeichereinheit ist eine Vielzahl von Zuordnungsregeln hinterlegt, wobei eine Zuordnungsregel eine Zuordnung zwischen mindestens einem Wertebereich für einen Patientenparameter und einer empfohlenen Geräteeinstellung für mindestens einen Beatmungsparameter des Beatmungsgerätes umfasst.

Die Verarbeitungseinheit ist ausgebildet, die statischen Patientendaten und die dynamischen Patientendaten zu empfangen und daraus jeweils unmittelbar nach dem Empfang entsprechender Patientendaten einen neuen aktuellen Patientendatensatz basierend auf den aktuell verfügbaren Patientendaten zu bestimmen, wobei die Verarbeitungseinheit weiterhin ausgebildet ist, ausgelöst durch das Bestimmen des neuen aktuellen Patientendatensatzes einen Vergleich zwischen den Werten des aktuellen Patientendatensatzes und den hinterlegten Zuordnungsregeln zu initiieren und basierend auf diesem Vergleich mindestens eine angesichts des aktuellen Patientendatensatzes empfohlene Geräteeinstellung des Beatmungsgerätes von der Regelspeichereinheit zu empfangen, und wobei die Verarbeitungseinheit ausgebildet ist, die mindestens eine empfohlene Geräteeinstellung mit einer aktuellen Geräteeinstellung des Beatmungsgerätes zu vergleichen und basierend auf diesem Vergleich eine Empfehlungsausgabe auszulösen, wobei die Empfehlungsausgabe die auf der mindestens einen empfohlenen Geräteeinstellung basierende Einstellungsempfehlung für den mindestens einen Beatmungsparameter indiziert.

Die Ausgabeeinheit ist ausgebildet, die Empfehlungsausgabe zu empfangen und die entsprechende Einstellungsempfehlung über ein Display grafisch auszugeben.

Im Rahmen der Erfindung wurde erkannt, dass eine Einstellungsempfehlung im klinischen Alltag schnell erfolgen muss. Daher wird mit dem Empfang von Patientendaten sofort durch die Verarbeitungseinheit geprüft, ob eine neue Geräteeinstellung empfohlen werden sollte. Insbesondere werden nicht erst dynamische Patientendaten für eine erste Empfehlung benötigt. Bereits nach dem ersten Empfang von Patientendaten, wie beispielsweise statischen Patientendaten wie Alter oder Gewicht, kann das medizinische System entsprechend der hinterlegten Zuordnungsregeln die Empfehlungsausgabe ausgeben.

Das Verwenden von bekannten Zuordnungsregeln, ermöglicht zudem ein nachvollziehbares Ableiten der Behandlungsempfehlung. So ermöglicht die unmittelbare Zuordnung zwischen dem Wertebereich eines Patientenparameters und der entsprechenden Geräteeinstellung über die Zuordnungsregel ein einfaches Verstehen der Funktionsweise des erfindungsgemäßen medizinischen Systems. Dies ist vorteilhaft zur Unterstützung eines Vertrauens des Anwenders in das System.

Die Bestandteile des erfindungsgemäßen medizinischen Systems können zumindest teilweise räumlich getrennt, insbesondere in verschiedenen Gehäusen angeordnet sein. Alternativ können auch alle Bestandteile des erfindungsgemäßen medizinischen Systems in einem gemeinsamen Gehäuse angeordnet sein. Die verschiedenen Bestandteile des erfindungsgemäßen medizinischen Systems sind zumindest auf Software-Ebene voneinander getrennt.

Geräte zum Bereitstellen von Patientendaten sind dem Fachmann auf diesem Gebiet bekannt, so dass neben einigen im Folgenden genannten Beispielen auch weitere Patientendaten und Geräte zum Bereitstellen solcher Patientendaten von dem medizinischen System genutzt werden können. Dabei erfolgt die Festlegung, bei welchen Patientendaten es sich um statische Patientendaten und bei welchen es sich um dynamische Patientendaten handelt vor einer Bereitstellung des erfindungsgemäßen medizinischen Systems. So gehört das Gewicht beispielsweise zu statischen Patientendaten, obwohl bei Neugeborenen eine sehr große Gewichtsschwankung möglich sein kann. Dieses Beispiel zeigt, dass auch ein regelmäßiges Empfangen von statischen aber eventuell veränderlichen Patientendaten sinnvoll sein kann. Messwertschnittstelle und Datenschnittstelle können gleich ausgebildet sein. Insbesondere können die beiden Schnittstellen als eine gemeinsame Schnittstelle ausgebildet sein, die sich lediglich auf Software-Ebene voneinander unterscheiden. Beide Schnittstellen ermöglichen den Empfang der Daten sowie deren Weiterverarbeitung durch die erfindungsgemäße Verarbeitungseinheit.

Der Vergleich zwischen der empfohlenen Geräteeinstellung und der aktuellen Geräteeinstellung kann beispielsweise der Vergleich eines empfohlenen Beatmungsparameters mit einem aktuell vorliegenden Beatmungsparameter sein. Beispielsweise kann in einer Ausführungsform die Empfehlungsausgabe nur ausgelöst werden, wenn der empfohlene Beatmungsparameter um mehr als 5%, insbesondere um mehr als 10%, von dem aktuell vorliegenden Beatmungsparameter abweicht. In einer weiteren Ausführungsform wird in diesem Vergleich ein Unterschied zwischen einem empfohlenen Beatmungsmodus und einem aktuell vorliegenden Beatmungsmodus erkannt und eine entsprechende Empfehlungsausgabe ausgelöst.

Nachfolgend werden bevorzugte Ausführungsformen des erfindungsgemäßen medizinischen Systems beschrieben.

Vorzugweise umfasst das erfindungsgemäße medizinische System weiterhin eine Benutzerschnittstelle, die ausgebildet ist, über eine Benutzereingabe eine anzuwendende Zuordnungsregel zu empfangen. Das Empfangen der anzuwendenden Zuordnungsregel über die Benutzerschnittstelle kann insbesondere ein Verändern einer angezeigten bereits existierenden Zuordnungsregel umfassen. Die Benutzereingabe kann dabei auf einem anderen Gerät erfolgen als der Empfang der Patientendaten.

In einer besonders bevorzugten Ausführungsform des medizinischen Systems führt der Vergleich zwischen der mindestens einen empfohlenen Geräteeinstellung und der aktuellen Geräteeinstellung des Beatmungsgerätes nur dann zum Auslösen der Empfehlungsausgabe, wenn ein empfohlener Stellwert für den mindestens einen Beatmungsparameters und ein aktuell vorliegender Stellwert des einzustellenden Beatmungsparameters eine vorbestimmte Stellwertmindestdifferenz voneinander haben. Hierdurch wird vorteilhaft sichergestellt, dass nicht bereits bei sehr kleinen Stellwertdifferenzen eine Empfehlungsausgabe dargestellt wird, die dann den Anwender von anderen wichtigen Informationen des Beatmungsgerätes ablenken könnte. Es ist anzunehmen, dass der Anwender bevorzugt nur dann an der Empfehlungsausgabe interessiert ist, wenn die empfohlene Veränderung der Geräteeinstellung eine ernsthafte Verbesserung der Behandlung bewirken könnte. Daher ist das Vorsehen der vorbestimmten Stellwertmindestdifferenz in dieser Ausführungsform besonders vorteilhaft um die Anzahl der Empfehlungsausgaben gering zu halten und mithin eine ernsthafte Auseinandersetzung des Anwenders mit der Empfehlungsausgabe zu erlauben. Die Stellwertmindestdifferenz kann ein vorbestimmter Absolutwert und/oder eine relative Abweichung zwischen empfohlenem Stellwert und vorliegendem Stellwert sein.

In einer besonders bevorzugten Ausführungsform indiziert die Empfehlungsausgabe zusätzlich die für die mindestens eine empfohlene Geräteeinstellung herangezogene Zuordnungsregel, wobei die Ausgabeeinheit weiter ausgebildet ist, die herangezogene Zuordnungsregel über das Display zumindest teilweise grafisch auszugeben. Hierdurch kann der Anwender besonders vorteilhaft den Grund für die Empfehlungsausgabe erkennen und dadurch entscheiden, ob er basierend auf der verwendeten Zuordnungsregel eine Veränderung der Geräteeinstellung vornehmen möchte. Hierdurch kann durch den Anwender kritisch der Grund für die Empfehlungsausgabe erkannt und beurteilt werden. So kann der Anwender die Empfehlungsausgabe beispielsweise durch individuell vorliegende Gründe ablehnen, wie beispielsweise: eine aktuelle Veränderung des Patientenzustands, wie etwa nach einem Aufwachen; eine persönliche Ablehnung der hinterlegten Zuordnungsregel; einen die Vorteile der empfohlenen Geräteeinstellung überwiegenden Effekt bei Beibehaltung der aktuellen Geräteeinstellung. Weiterhin vorteilhaft an der vorliegenden Ausführungsform ist das unterstützte Vertrauen in die Empfehlungsausgabe, da der Anwender transparent erkennen kann, warum es zu dieser Empfehlungsausgabe kam. Hierdurch kann besonders gut bewertet werden, ob der Empfehlungsausgabe gefolgt werden sollte oder nicht. Zudem erlaubt die Ausgabe in dieser Ausführungsform ein schnelles Erkennen eines veränderten Zustands der Behandlung, welcher eventuell zu der Empfehlungsausgabe geführt hat.

In einer vorteilhaften Variante der vorhergehenden Ausführungsform ist die Verarbeitungseinheit weiter ausgebildet, mindestens zwei empfohlene Geräteeinstellungen mit der entsprechenden, herangezogenen Zuordnungsregel über die Empfehlungsausgabe auszugeben, falls für diese mindestens zwei empfohlenen Geräteeinstellungen der entsprechend empfohlene Stellwert und der aktuell vorliegende Stellwert die jeweilige vorbestimmte Stellwertmindestdifferenz voneinander haben, und wobei die Ausgabeeinheit ausgebildet ist, für die mindestens zwei empfohlenen Geräteeinstellungen neben der Einstellungsempfehlung auch die herangezogenen Zuordnungsregeln über das Display zumindest teilweise grafisch auszugeben. In dieser Variante wird sichergestellt, dass der Anwender selbst zwischen zwei empfohlenen Geräteeinstellungen oder dem Beibehalten der aktuellen Einstellung entscheiden kann. Diese Variante verdeutlicht, dass das erfindungsgemäße medizinische System keine Entscheidung für den Anwender trifft, sondern verschiedene Empfehlungen sowie deren Gründe in Form der Zuordnungsregeln ungefiltert ausgibt. So kann der Anwender selbst entscheiden, wie die Behandlung weiter verlaufen sollte und/oder welche angewendete Zuordnungsregel die größere Relevanz für die aktuelle Behandlung hat.

In einer bevorzugten Ausführungsform ist die Verarbeitungseinheit bei Beginn einer Beatmung durch das Beatmungsgerät ausgebildet, unmittelbar nach dem ersten empfangenen Wert eines Patientenparameters den aktuellen Patientendatensatz zu bestimmen und abhängig von dem Vergleich zwischen empfohlener Geräteeinstellung und aktueller Geräteeinstellung die Empfehlungsausgabe auszulösen. In dieser Ausführungsform wird besonders schnell eine erste Empfehlungsausgabe bereitgestellt, so dass das medizinische System bereits zu Beginn einer Behandlung durch eine Empfehlungsausgabe helfen könnte.

In einer vorteilhaften Variante der vorhergehenden Ausführungsform ist die Verarbeitungseinheit bei Beginn einer Beatmung durch das Beatmungsgerät ausgebildet, entsprechend einer vorbestimmten Bearbeitungshierarchie empfangene statische Patientendaten zeitlich vor empfangenen dynamischen Patientendaten für die Bestimmung des aktuellen Patientendatensatzes zu berücksichtigen. Statische Patientendaten können besonders einfach und unmittelbar ausgewertet werden. Daher erlaubt diese Variante ein besonders schnelles Bereitstellen einer ersten Einstellungsempfehlung, welche dann etwas später durch die Berücksichtigung der dynamischen Patientendaten eventuell wieder geändert wird. Hierdurch wird sichergestellt, dass die Empfehlungsausgabe nicht erst erfolgt, wenn eine Vielzahl von Patientendaten bestimmt und ausgewertet wurden. Zudem wird sichergestellt, dass auch bei noch nicht aktivierten Komponenten des verwendeten Beatmungssystems, wie beispielsweise einem noch nicht ordnungsgemäß angebrachten Sensor, sofort eine Einstellungsempfehlung basierend auf statischen Patientendaten empfangen werden kann, um einen sinnvollen und effizienten Start der Behandlung sicherzustellen.

Vorzugsweise betreffen die statischen Patientendaten eine Gruppe von Patientenparametern, die mindestens einen der folgenden Parameter umfasst: Patientenkategorie; Größe; Alter; Geschlecht; Gewicht; Beatmungsmodus; Vorerkrankung. Derartige Patientendaten werden typischerweise zu Beginn einer Behandlung aufgenommen und verändern sich wenig im Verlauf einer Behandlung.

Vorzugsweise betreffen die dynamischen Patientendaten eine Gruppe von Patientenparametern, die mindestens einen der folgenden Parameter umfasst: Sensordaten der Beatmung; Diagnosedaten; Blutgaswerte; Medikation; hämodynamische Kennzahl; Bettstellung; aktuelles Beatmungsmanöver; aus Sensordaten abgeleitete Daten wie etwa Tidalvolumen und/oder Minutenvolumen. Derartige Patientendaten werden typischerweise wiederholt, insbesondere im Wesentlichen kontinuierlich aufgenommen. Derartige sich schnell verändernde Patientendaten sorgen im Rahmen der vorliegenden Erfindung während der Behandlung für das Ausgeben einer Einstellungsempfehlung, wohingegen statische Patientendaten typischerweise zu Beginn einer Behandlung eine große Relevanz für die Ausgabe der Einstellungsempfehlung haben.

In einer besonders bevorzugten Ausführungsform sind die hinterlegten Zuordnungsregeln in Form einer "Wenn-Dann"-Fallunterscheidung ausgebildet. Eine derartige Form der Zuordnungsregeln erlaubt ein besonders einfaches Erfassen der Gründe für die Empfehlungsausgabe, insbesondere wenn die angewendete Zuordnungsregel mit ausgegeben wird. Derartige einfach strukturierte Zuordnungsregeln ermöglichen eine einfache Eingabe und/oder Korrektur der Zuordnungsregeln über die Benutzerschnittstelle. Zudem reduziert solch eine einfache Struktur der Zuordnungsregeln das Risiko einer fehlerhaft hinterlegten Zuordnungsregel.

In einer weiteren bevorzugten Ausführungsform wird das Verarbeiten der Patientendaten durch das medizinische System ausgelöst durch einen Empfang eines Triggersignals. Ein solches Triggersignal kann beispielsweise eine Aktivierung des erfindungsgemäßen medizinischen Systems anzeigen. Alternativ oder ergänzend kann der Start der Behandlung, beispielsweise durch den Empfang erster Patientendaten, das Triggersignal auslösen und dadurch die Verarbeitung der Patientendaten zum Bereitstellen einer Einstellungsempfehlung aktivieren. Hierdurch kann daher sichergestellt werden, dass die Empfehlungsausgaben automatisiert ab einem vorbestimmten und für den Anwender nachvollziehbaren Zeitpunkt ausgegeben werden.

In einer besonders vorteilhaften Ausführungsform ist die Benutzerschnittstelle weiterhin ausgebildet, eine durch einen Anwender des medizinischen Systems ausgelöste Annahme einer empfohlenen Geräteeinstellung zu empfangen und ein entsprechendes Annahmesignal an das Beatmungsgerät auszugeben. In dieser Ausführungsform kann besonders einfach und benutzerfreundlich die aktuelle Geräteeinstellung des Beatmungsgerätes entsprechend der Einstellungsempfehlung verändert werden. Insbesondere bei einer empfohlenen Verstellung mehrerer Stellwerte kann so durch eine einzige Benutzerinteraktion die Behandlung des Patienten an die empfohlene Behandlung angepasst werden.

Vorzugsweise weist das erfindungsgemäße medizinische System weiterhin das Beatmungsgerät auf, wobei das Beatmungsgerät vorliegende Beatmungsgerätedaten über die Ausgabeeinheit an einen Anwender des medizinischen Systems ausgibt. Die erfindungsgemäße Ausgabeeinheit ist folglich die gleiche Einheit, die auch die üblichen Beatmungsgerätedaten ausgibt. Daher erlaubt diese Ausführungsform vorteilhaft die Vermeidung mehrerer Displays und folglich eine einfache Nutzbarkeit des medizinischen Systems. Unter einem Beatmungsgerät ist im Rahmen dieser Erfindung ein medizinisches Gerät zur Beatmung eines Patienten zu verstehen. So ist neben einem klassischen Beatmungsgerät beispielsweise auch ein Anästhesiegerät ein Beatmungsgerät im Sinne dieser Erfindung und kann folglich ebenfalls Bestandteil des erfindungsgemäßen medizinischen Systems sein.

Vorzugsweise ist die Verarbeitungseinheit ausgebildet, nach einer vorbestimmten Wartezeit, in der keine Patientendaten empfangen wurden, eine Datenanfrage an ein verbundenes medizinisches Gerät auszugeben. In dieser Ausführungsform wird vermieden, dass aufgrund eines nicht erkannten Fehlers, wie beispielswiese eines Verbindungsproblems zwischen zwei Geräten, keine erfindungsgemäße Empfehlungsausgabe ermöglicht wird. Die Datenanfrage kann beispielsweise zur Aufforderung an den Anwender des Systems führen, bestimmte statische Patientendaten erneut oder erstmalig einzugeben und/oder die Verbindung mit einem bestimmten Gerät, wie beispielsweise mit einem Sensor zum Erfassen dynamischer Patientendaten, zu überprüfen. Daher reduziert das medizinische System gemäß dieser Ausführungsform eine Wahrscheinlichkeit einer unerkannten Fehlfunktion des Systems und/oder aufgrund einer Fehlbedienung durch den Anwender.

Gemäß einem weiteren Aspekt der Erfindung wird zur Lösung der oben genannten Aufgabe ein Verfahren zur Bereitstellung einer Einstellungsempfehlung für ein Beatmungsgerät für die Beatmung eines Patienten vorgeschlagen. Das erfindungsgemäße Verfahren weist die folgenden Schritte auf:
- Hinterlegen einer Vielzahl von Zuordnungsregeln, wobei eine Zuordnungsregel eine Zuordnung zwischen mindestens einem Wertebereich für einen Patientenparameter und einer empfohlenen Geräteeinstellung für mindestens einen Beatmungsparameter des Beatmungsgerätes umfasst;
- Empfangen statischer Patientendaten, wobei statische Patientendaten Werte von Patientenparametern umfassen, die im Laufe einer Behandlungsdauer im Wesentlichen unveränderlich sind;
- Empfangen dynamischer Patientendaten, wobei dynamische Patientendaten Werte von Patientenparametern umfassen, die sich im Laufe der Behandlungsdauer verändern;
- Bestimmen eines neuen aktuellen Patientendatensatzes basierend auf den aktuell verfügbaren Patientendaten unmittelbar nach dem Empfang entsprechender Patientendaten;
- Initiieren eines Vergleichs zwischen den Werten des aktuellen Patientendatensatzes und den hinterlegten Zuordnungsregeln, ausgelöst durch das Bestimmen des neuen aktuellen Patientendatensatzes;
- Empfangen einer angesichts des aktuellen Patientendatensatzes empfohlenen Geräteeinstellung des Beatmungsgerätes;
- Vergleichen der mindestens einen empfohlenen Geräteeinstellung mit einer aktuellen Geräteeinstellung des Beatmungsgerätes und Auslösen einer Empfehlungsausgabe basierend auf diesem Vergleich, wobei die Empfehlungsausgabe die auf der mindestens einen empfohlenen Geräteeinstellung basierende Einstellungsempfehlung für den mindestens einen Beatmungsparameter indiziert;
- Empfangen der Empfehlungsausgabe und grafische Ausgabe der entsprechenden Einstellungsempfehlung.

Dieses Verfahren wird von dem erfindungsgemäßen medizinischen System ausgeführt und hat daher sämtliche Vorteile dieses Systems. Insbesondere erlaubt es eine zuverlässige, nachvollziehbare und schnelle Ausgabe der Einstellungsempfehlung.

Die Schritte des erfindungsgemäßen Verfahrens können zumindest teilweise auf einem gemeinsamen Prozessor oder auf räumlich voneinander getrennten Komponenten ausgeführt werden.

Vorzugsweise werden entsprechend einer vorbestimmten Bearbeitungshierarchie für die Bestimmung des aktuellen Patientendatensatzes empfangene statische Patientendaten zeitlich vor empfangenen dynamischen Patientendaten berücksichtigt. Hierdurch wird eine besonders schnelle Ausgabe der Einstellungsempfehlung basierend auf den wenig veränderlichen statische Patientendaten ermöglicht, ehe möglicherweise basierend auf den dynamischen Patientendaten eine Korrekter dieser Einstellungsempfehlung zu einem späteren Zeitpunkt erfolgt.

Die Erfindung soll nun anhand von in den Figuren schematisch dargestellten, vorteilhaften Ausführungsbeispielen näher erläutert werden. Von diesen zeigen im Einzelnen:
- Fig. 1: eine schematische Darstellung eines ersten Ausführungsbeispiels eines erfindungsgemäßen medizinischen Systems;
- Fig. 2: eine schematische Darstellung eines zweiten Ausführungsbeispiels des erfindungsgemäßen medizinischen Systems;
- Fig. 3: eine schematische Darstellung eines dritten Ausführungsbeispiels des erfindungsgemäßen medizinischen Systems;
- Fig. 4: ein Flussdiagramm eines Ausführungsbeispiels eines Verfahrens gemäß einem weiteren Aspekt der Erfindung.

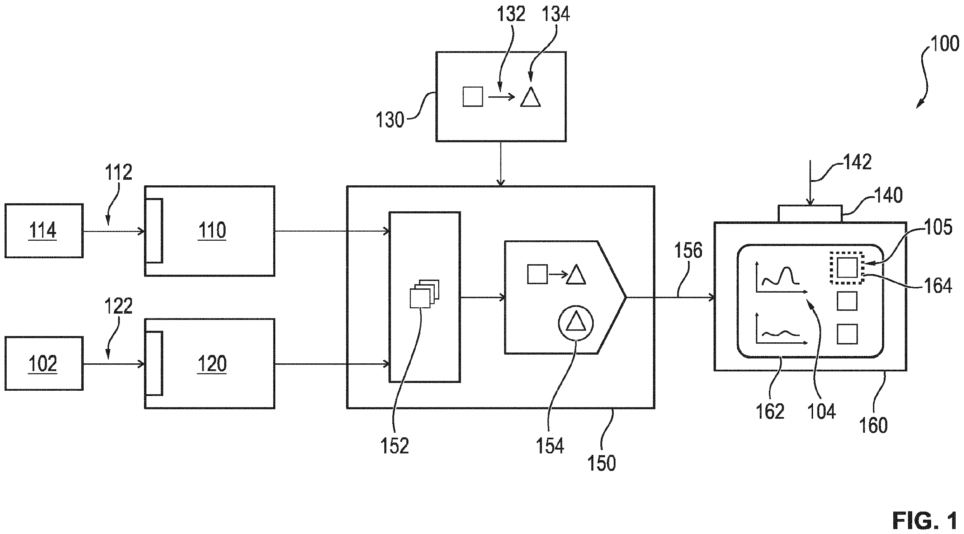

Fig. 1 zeigt eine schematische Darstellung eines ersten Ausführungsbeispiels eines erfindungsgemäßen medizinischen Systems 100.

Das medizinische System 100 ist zur Bereitstellung einer Einstellungsempfehlung 105 für ein Beatmungsgerät 102 für die Beatmung eines Patienten ausgebildet. Das medizinische System 100 weist hierfür zumindest eine Datenschnittstelle 110, eine Messwertschnittstelle 120, eine Regelspeichereinheit 130, eine Verarbeitungseinheit 150 und eine Ausgabeeinheit 160 auf. Zusätzlich weist das medizinische System 100 in dem dargestellten Ausführungsbeispiel eine Benutzerschnittstelle 140 auf. Diese Bestandteile des medizinischen Systems 100 können beabstandet voneinander oder zumindest teilweise in einem gemeinsamen Gehäuse angeordnet sein. Die Unterteilung zwischen den verschiedenen Bestandteilen erfolgt dabei zumindest auf Software-Ebene.

Die Datenschnittstelle 110 ist ausgebildet, statische Patientendaten 112 zumindest aus einer Patientendatenbank 114 zu empfangen, wobei statische Patientendaten 112 Werte von Patientenparametern umfassen, die im Laufe einer Behandlungsdauer im Wesentlichen unveränderlich sind.

Die Messwertschnittstelle 120 ist ausgebildet, dynamische Patientendaten 122 zumindest von einem Beatmungsgerät 102 zu empfangen, wobei dynamische Patientendaten 122 Werte von Patientenparametern umfassen, die sich im Laufe der Behandlungsdauer verändern. Die beiden Schnittstellen 110, 120 sind vorliegend als drahtlose Schnittstellen ausgebildet, wie beispielsweise als WLAN, Bluetooth, BLE, ZigBee-Schnittstelle oder dergleichen. Alternativ oder ergänzend kann mindestens eine der beiden Schnittstellen als kabelgebundene Schnittstelle ausgebildet sein. Die beiden Schnittstellen können alternativ auch als gemeinsame Schnittstelle ausgebildet sein, die während des Empfangs von statischen Patientendaten als Messwertschnittstelle und beim Empfang von dynamischen Patientendaten als Datenschnittstelle fungiert. Der Aufbau derartiger Schnittstellen ist dem Fachmann grundsätzlich bekannt, so dass darauf im Folgenden nicht weiter eingegangen werden wird.

In der Regelspeichereinheit 130 ist eine Vielzahl von Zuordnungsregeln 132 hinterlegt. Eine jeweilige Zuordnungsregel 132 umfasst dabei eine Zuordnung zwischen mindestens einem Wertebereich für einen Patientenparameter und einer empfohlenen Geräteeinstellung 134 für mindestens einen Beatmungsparameter des Beatmungsgerätes 102. Die Regelspeichereinheit 130 ist zumindest indirekt mit der Benutzerschnittstelle 140 verbunden, was in Fig. 1 aus Gründen der Übersichtlichkeit nicht dargestellt ist.

Die Benutzerschnittstelle 140 ist ausgebildet, über eine Benutzereingabe 142 eine anzuwendende Zuordnungsregel 132 zu empfangen. Die anzuwendende Zuordnungsregel kann beispielsweise eine angepasste ursprünglich hinterlegte Zuordnungsregel, eine neu hinterlegte Zuordnungsregel oder eine Auswahl aus einer Gruppe von möglichen hinterlegten Zuordnungsregeln umfassen. In dem dargestellten Ausführungsbeispiel ist die Benutzerschnittstelle 140 an der Ausgabeeinheit 160 angeordnet. Hierbei ist das Display 162 der Ausgabeeinheit 160 ein Touch-Display, welches in Kombination mit möglichen weiteren Tasten die Benutzerschnittstelle 140 bildet, um die entsprechende Benutzereingabe 142 zu empfangen. In einem alternativen oder ergänzenden Ausführungsbeispiel des erfindungsgemäßen medizinischen Systems ist keine derartige Benutzerschnittstelle zur Eingabe einer Zuordnungsregel vorgesehen, da beispielsweise sämtliche Zuordnungsregeln bei der Herstellung des Systems oder bei Wartungsarbeiten an dem System verändert, angepasst und/oder bereitgestellt werden.

Die Verarbeitungseinheit 150 ist ausgebildet, die statischen Patientendaten 112 und die dynamischen Patientendaten 122 zu empfangen und daraus jeweils unmittelbar nach dem Empfang entsprechender Patientendaten einen neuen aktuellen Patientendatensatz 152 basierend auf den aktuell verfügbaren Patientendaten 112, 122 zu bestimmen. Dabei ist die Verarbeitungseinheit 150 weiterhin ausgebildet, ausgelöst durch das Bestimmen des neuen aktuellen Patientendatensatzes 152 einen Vergleich zwischen den Werten des aktuellen Patientendatensatzes 152 und den hinterlegten Zuordnungsregeln 132 zu initiieren und basierend auf diesem Vergleich mindestens eine angesichts des aktuellen Patientendatensatzes 152 empfohlene Geräteeinstellung 134 des Beatmungsgerätes 132 von der Regelspeichereinheit 130 zu empfangen. Zudem führt die Verarbeitungseinheit 150 einen Vergleich aus, bei dem die mindestens eine empfohlene Geräteeinstellung 134 mit einer aktuellen Geräteeinstellung 154 des Beatmungsgerätes 102 verglichen wird. Basierend auf diesem Vergleich wird eine Empfehlungsausgabe 156 ausgelöst, wobei die Empfehlungsausgabe 156 die auf der mindestens einen empfohlenen Geräteeinstellung basierende Einstellungsempfehlung 105 für den mindestens einen Beatmungsparameter indiziert.

Die Ausgabeeinheit 160 ist vorliegend ein Touch-Display, welches die empfangene Empfehlungsausgabe 156 grafisch über die Hervorhebung eines Auswahlbereiches 164 auf dem Touch-Display ausgibt. Die dargestellte Hervorhebung durch eine Umrandung ist lediglich beispielhaft. In anderen Ausführungsbeispielen wird ein separates Auswahlfenster auf der Benutzeroberfläche des Beatmungsgerätes als Empfehlungsausgabe verwendet.

Ein Beatmungsgerät im Sinne der Erfindung ist ein Gerät zur Beatmung eines Patienten, wie etwa auch ein Anästhesiegerät. Für ein Anästhesiegerät kann das erfindungsgemäße medizinische System daher auch verwendet werden, um eine Einstellungsempfehlung auszugeben.

Die Einstellungsempfehlung kann erfindungsgemäß eine Verstellung einer Anzahl von Stellwerten von Beatmungsparametern und/oder ein Wechseln oder Aktivieren eines konkreten Beatmungsmodus anzeigen.

Die Beatmungsgerätedaten 104 werden in dem dargestellten Ausführungsbeispiel ebenfalls über das Display der Ausgabeeinheit 160 ausgegeben. Hierdurch muss ein Anwender nicht mehrere Displays kontrollieren, um auf die Ausgaben des erfindungsgemäßen medizinischen Systems aufmerksam zu werden.

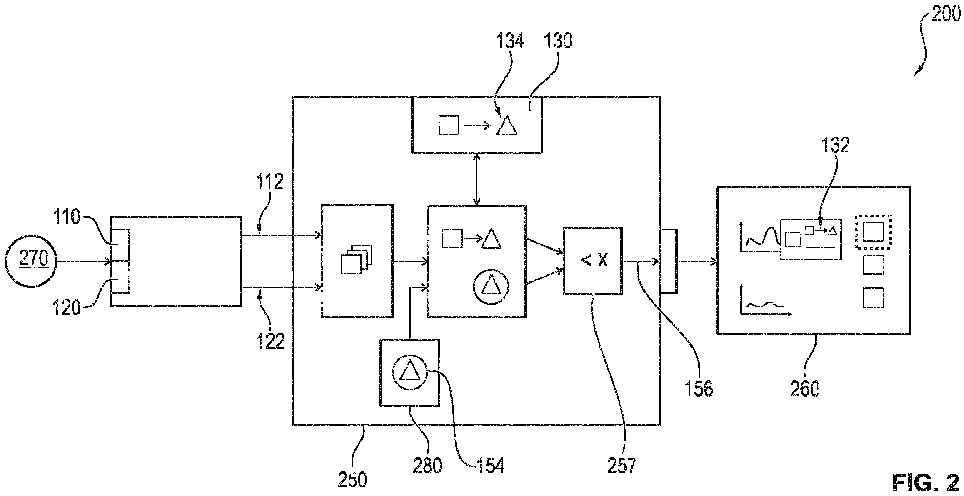

Fig. 2 zeigt eine schematische Darstellung eines zweiten Ausführungsbeispiels des erfindungsgemäßen medizinischen Systems 200.

Das medizinische System 200 unterscheidet sich von dem in Fig. 1 dargestellten System 100 unter anderem dadurch, dass die Datenschnittstelle 110 und die Messwertschnittstelle 120 in einem gemeinsamen Gehäuse angeordnet sind und lediglich auf Software-Ebene voneinander getrennt sind. So empfangen die Schnittstellen beide über eine drahtlose Verbindung mit einem Netzwerk 270, insbesondere ein Krankenhaus-Netzwerk, die entsprechenden Patientendaten. Alternativ oder ergänzend kann die Verbindung mit dem Netzwerk drahtgebunden sein. Die beiden dargestellten Signale für die Übertragung der statischen Patientendaten 112 und der dynamischen Patientendaten 122 können auch über ein kombiniertes Signal an die Verarbeitungseinheit 250 bereitgestellt werden.

Die Verarbeitungseinheit 250 ist unmittelbar verbunden mit der Regelspeichereinheit 130 ausgebildet. Zudem verfügt die Verarbeitungseinheit über einen internen Speicher 280, auf dem stets die aktuell vorliegende Geräteeinstellung 154 für den Vergleich mit der empfohlenen Geräteeinstellung 134 hinterlegt ist.

Dieser Vergleich erfolgt vorliegend derart, dass nur dann eine Empfehlungsausgabe 156 ausgegeben wird, wenn ein empfohlener Stellwert für den mindestens einen Beatmungsparameters und ein aktuell vorliegender Stellwert des einzustellenden Beatmungsparameters eine vorbestimmte Stellwertmindestdifferenz 257 voneinander haben. Diese vorbestimmte Stellwertmindestdifferenz 257 ist vorliegend mindestens 2% des aktuell vorliegenden Stellwerts, insbesondere mindestens 4% des aktuell vorliegenden Stellwerts, besonders bevorzugt 6% des aktuell vorliegenden Stellwerts.

Weiterhin unterscheidet sich die Ausgabeeinheit 260 von der Ausgabeeinheit 160 aus Fig. 1 darin, dass die für die Empfehlungsausgabe 156 herangezogene Zuordnungsregel 132 über das Display der Ausgabeeinheit 260 grafisch ausgegeben wird. Hierdurch kann der Anwender schnell erkennen, warum es zu der angezeigten empfohlenen Geräteeinstellung 134 gekommen ist. Besonders vorteilhaft ist das dargestellte Ausführungsbeispiel des medizinischen Systems 200 dazu ausgebildet, bei dem Ausgeben von zwei verschiedenen empfohlenen Geräteeinstellungen für beide die der Empfehlung zugrundeliegende Zuordnungsregel mit auszugeben. Hierdurch wird nicht intransparent zwischen zwei gleichberechtigten Einstellungsempfehlungen ohne Eingriff des Anwenders eine herausgefiltert, sondern eine Auswahl durch den informierten Anwender ermöglicht. Dies unterstreicht einen wesentlichen Aspekt der vorliegenden Erfindung, der darin liegt, dass durch das medizinische System keine Entscheidungen automatisiert getroffen werden sondern lediglich Empfehlungen an den Anwender ausgegeben werden, die in diesem Beispiel vorteilhaft mit den Hintergründen der Empfehlung, nämlich der Zuordnungsregel, unterstützt werden.

Vorteilhaft ist das dargestellte medizinische System 200 dazu ausgebildet, mit dem Empfang der ersten Patientendaten zu Beginn einer Behandlung den Vergleich zwischen empfohlener Geräteeinstellung und aktueller Geräteeinstellung auszuführen und gegebenenfalls die Empfehlungsausgabe 156 auszugeben. Hierfür weist die Verarbeitungseinheit 250 eine Bearbeitungshierarchie der Patientendaten auf, gemäß welcher empfangene stationäre Patientendaten zeitlich vor empfangenen dynamischen Patientendaten für die Bestimmung des aktuellen Patientendatensatzes berücksichtigt werden. So kann vorteilhaft sehr schnell eine auf stationären Patientendaten basierende Empfehlungsausgabe bereitgestellt werden, die auf eine anfänglich fehlerhafte Einstellung des Beatmungsgerätes direkt aufmerksam machen kann. So wird vermieden, dass erst eine große Anzahl von Daten gesammelt werden muss, ehe eine Empfehlung durch das System möglich wird.

Statische Patientendaten im Sinne dieser Erfindung können beispielsweise sein: Patientenkategorie; Größe; Alter; Geschlecht; Gewicht; Beatmungsmodus; Vorerkrankungen.

Dynamische Patientendaten im Sinne dieser Erfindung können beispielsweise sein: Sensordaten der Beatmung; Diagnosedaten; Blutgaswerte; Medikation; hämodynamische Kennzahl; Bettstellung; aktuelles Beatmungsmanöver; aus Sensordaten abgeleitete Daten wie etwa Tidalvolumen und/oder Minutenvolumen.

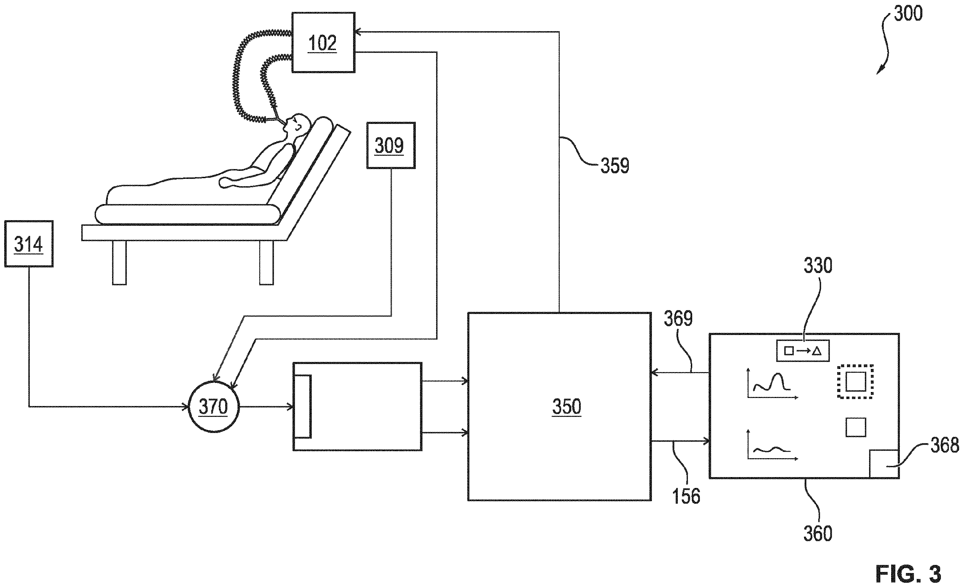

Fig. 3 zeigt eine schematische Darstellung eines dritten Ausführungsbeispiels des erfindungsgemäßen medizinischen Systems 300.

Das dargestellte medizinische System 300 unterscheidet sich dadurch von dem in Fig. 2 dargestellten medizinischen System 200, dass auf dem Display der Ausgabeeinheit 360 ein Trigger-Bereich 368 angezeigt wird, durch den bei Berührung die erfindungsgemäße Verarbeitung der Patientendaten ausgelöst werden kann. Hierdurch kann der Anwender vorteilhaft selbst entscheiden, ob eine Empfehlungsausgabe 156 ausgegeben wird oder nicht.

Die Empfehlungsausgabe 156 wird vorliegend, wie bereits in Fig. 2 gezeigt, als Ausgabefenster mit der aktuell zur Empfehlung führenden Zuordnungsregel ausgegeben. Bei den Zuordnungsregeln aus der nicht dargestellten Regelspeichereinheit handelt es sich vorliegend um "Wenn-Dann"-Fallunterscheidungen. So kann eine Zuordnungsregel beispielsweise lauten, dass wenn ein Messwert eines Patientenparameters einen vorbestimmten Schwellenwert übersteigt, ein vorbestimmter Beatmungsmodus aktiviert sein soll. Wenn dieser Modus als bisher nicht aktiviert von der Verarbeitungseinheit 350 erkannt wird, würde dies entsprechend zu einer Empfehlungsausgabe 156 führen, dass dieser Beatmungsmodus angesichts der entsprechenden Zuordnungsregel aktiviert werden sollte. Alternativ oder ergänzend können die verwendeten Zuordnungsregeln sich aus sogenannten Look-Up-Tabellen ergeben, die beispielsweise als interne Richtlinie für bestimmte Behandlungen bekannt sind.

Weiterhin ist die an der Ausgabeeinheit 360 angeordnete Benutzerschnittstelle 330 ausgebildet, über einen entsprechenden Eingabebereich eine durch einen Anwender des medizinischen Systems ausgelöste Annahme einer empfohlenen Geräteeinstellung zu empfangen und ein entsprechendes Annahmesignal 369 an das Beatmungsgerät auszugeben. Diese Ausgabe ist vorliegend lediglich als ein Annahmesignal an die Verarbeitungseinheit 350 dargestellt. Der Weg des Annahmesignals 369 von der Verarbeitungseinheit weiter zu dem Beatmungsgerät ist aus Gründen der Übersichtlichkeit nicht dargestellt. Das Annahmesignal 369 kann von der Verarbeitungseinheit 350 direkt oder über ein weiteres Gerät zu dem Beatmungsgerät geführt werden. In einem nicht dargestellten Ausführungsbeispiel wird das Annahmesignal direkt an das Beatmungsgerät von der Benutzerschnittstelle oder der Ausgabeeinheit ausgegeben.

Schließlich ist die Verarbeitungseinheit 350 auch ausgebildet, nach einer vorbestimmten Wartezeit, in der keine Patientendaten empfangen wurden, eine Datenanfrage 359 an ein verbundenes medizinisches Gerät auszugeben. Vorliegend erfolgt die Datenanfrage über das Netzwerk 370, insbesondere das Krankenhaus-Netzwerk. Welches Gerät die entsprechende Datenanfrage 359 empfängt, ist abhängig von dem Patientenparameter, von dem innerhalb der Wartezeit keine Daten empfangen wurden. Sollten beispielsweise innerhalb einer vorbestimmten Wartezeit von mindestens 10 min, insbesondere mindestens 20min nach Beginn der Behandlung eines Patienten keine Angaben zu seinem Alter empfangen worden sein, wird die Datenanfrage 359 an die Patientendatenbank 314 gesendet. Sollten hingegen beispielsweise innerhalb einer vorbestimmten Wartezeit von mindestens 20 Sekunden, insbesondere mindestens 1 Minute nach dem letzten Empfang dieser Daten keine Gasfluss-Daten empfangen worden sein, wird die Datenanfrage 359 an die Sensoreinheit 309 oder das Beatmungsgerät 102 gesendet.

In dem dargestellten Ausführungsbeispiel sind alle patientennahen Geräte mit dem Netzwerk 370 verbunden und kommunizieren über dieses Netzwerk mit dem erfindungsgemäßen medizinischen System 300. Alternativ oder ergänzend können Geräte auch zumindest teilweise direkt mit dem medizinischen System verbunden sein.

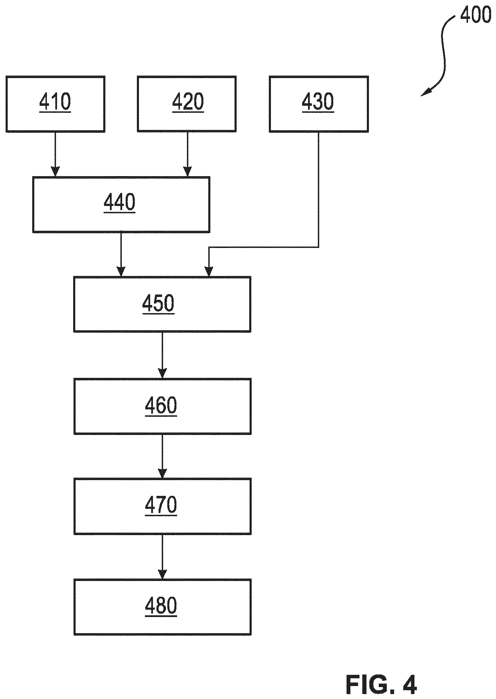

Fig. 4 zeigt ein Flussdiagramm eines Ausführungsbeispiels eines Verfahrens 400 gemäß einem weiteren Aspekt der Erfindung.

Das erfindungsgemäße Verfahren 400 ist zur Bereitstellung einer Einstellungsempfehlung für ein Beatmungsgerät für die Beatmung eines Patienten ausgebildet. Hierfür weist es die im Folgenden dargestellten Verfahrensschritte auf.

Ein erster Schritt 410 umfasst ein Hinterlegen einer Vielzahl von Zuordnungsregeln, wobei eine Zuordnungsregel eine Zuordnung zwischen mindestens einem Wertebereich für einen Patientenparameter und einer empfohlenen Geräteeinstellung für mindestens einen Beatmungsparameter des Beatmungsgerätes umfasst.

Ein weiterer Schritt 420 umfasst ein Empfangen statischer Patientendaten, wobei statische Patientendaten Werte von Patientenparametern umfassen, die im Laufe einer Behandlungsdauer im Wesentlichen unveränderlich sind.

Ein weiterer Schritt 430 umfasst ein Empfangen dynamischer Patientendaten, wobei dynamische Patientendaten Werte von Patientenparametern umfassen, die sich im Laufe der Behandlungsdauer verändern.

Ein darauffolgender Schritt 440 umfasst ein Bestimmen eines neuen aktuellen Patientendatensatzes basierend auf den aktuell verfügbaren Patientendaten unmittelbar nach dem Empfang entsprechender Patientendaten.

Ein nächster Schritt 450 umfasst ein Initiieren eines Vergleichs zwischen den Werten des aktuellen Patientendatensatzes und den hinterlegten Zuordnungsregeln, ausgelöst durch das Bestimmen des neuen aktuellen Patientendatensatzes.

Ein weiterer Schritt 460 umfasst ein Empfangen einer angesichts des aktuellen Patientendatensatzes empfohlenen Geräteeinstellung des Beatmungsgerätes.

Ein darauffolgender Schritt 470 umfasst ein Vergleichen der mindestens einen empfohlenen Geräteeinstellung mit einer aktuellen Geräteeinstellung des Beatmungsgerätes und ein Auslösen einer Empfehlungsausgabe basierend auf diesem Vergleich, wobei die Empfehlungsausgabe die auf der mindestens einen empfohlenen Geräteeinstellung basierende Einstellungsempfehlung für den mindestens einen Beatmungsparameter indiziert.

Ein abschließender Schritt 480 umfasst ein Empfangen der Empfehlungsausgabe und grafische Ausgabe der entsprechenden Einstellungsempfehlung.

Die Schritte 410, 420 und 430 können unabhängig voneinander erfolgen. Insbesondere das Hinterlegen der Zuordnungsregeln gemäß Schritt 410 kann zeitlich vor den weiteren Schritten ausgeführt werden, beispielsweise vor dem Einsatz des medizinischen Systems. Die weiteren Verfahrensschritte basieren zumindest teilweise aufeinander und werden daher vorzugweise in der dargestellten Reihenfolge ausgeführt.

In einem besonders bevorzugten Ausführungsbeispiel werden entsprechend einer vorbestimmten Bearbeitungshierarchie für die Bestimmung des aktuellen Patientendatensatzes empfangene statische Patientendaten zeitlich vor empfangenen dynamischen Patientendaten berücksichtigt. Hierdurch kann besonders schnell eine erfindungsgemäße Einstellungsempfehlung bereitgestellt werden. Vorzugsweise erfolgt die Ausgabe der Einstellungsempfehlung weniger als 20 Sekunden, insbesondere weniger als 10 Sekunden, besonders bevorzugt weniger als 5 Sekunden nach dem Empfang der ersten statischen Patientendaten.

### Bezugszeichenliste

- 100, 200, 300: medizinisches System
- 102: Beatmungsgerät
- 104: Beatmungsgerätedaten
- 105: Einstellungsempfehlung
- 110: Datenschnittstelle
- 112: statische Patientendaten
- 114, 314: Patientendatenbank
- 120: Messwertschnittstelle
- 122: dynamische Patientendaten
- 130, 330: Regelspeichereinheit
- 132: Zuordnungsregel
- 134: empfohlene Geräteeinstellung
- 140: Benutzerschnittstelle
- 142: Benutzereingabe
- 150, 250, 350: Verarbeitungseinheit
- 152: aktueller Patientendatensatz
- 154: aktuelle Geräteeinstellung
- 156: Empfehlungsausgabe
- 160, 260, 360: Ausgabeeinheit
- 162: Display
- 164: hervorgehobener Auswahlbereich
- 257: vorbestimmte Stellwertmindestdifferenz
- 270, 370: Netzwerk
- 280: interner Speicher
- 309: Sensoreinheit
- 359: Datenanfrage
- 368: Trigger-Bereich
- 369: Annahmesignal
- 400: Verfahren
- 410, 420, 430, 440, 450: Verfahrensschritte
460, 470, 480

## Patentansprüche

1. Medizinisches System (100) zur Bereitstellung einer Einstellungsempfehlung (105) für ein Beatmungsgerät (102) für die Beatmung eines Patienten, mit
- einer Datenschnittstelle (110), die ausgebildet ist, statische Patientendaten (112) zumindest aus einer Patientendatenbank (114) zu empfangen, wobei statische Patientendaten (112) Werte von Patientenparametern umfassen, die im Laufe einer Behandlungsdauer im Wesentlichen unveränderlich sind,
- einer Messwertschnittstelle (120), die ausgebildet ist, dynamische Patientendaten (122) zumindest von einem Beatmungsgerät (102) zu empfangen, wobei dynamische Patientendaten (122) Werte von Patientenparametern umfassen, die sich im Laufe der Behandlungsdauer verändern,
- einer Regelspeichereinheit (130), in der eine Vielzahl von Zuordnungsregeln (132) hinterlegt ist, wobei eine Zuordnungsregel (132) eine Zuordnung zwischen mindestens einem Wertebereich für einen Patientenparameter und einer empfohlenen Geräteeinstellung (134) für mindestens einen Beatmungsparameter des Beatmungsgerätes (102) umfasst,
- einer Verarbeitungseinheit (150), die ausgebildet ist, die statischen Patientendaten (112) und die dynamischen Patientendaten (122) zu empfangen und daraus jeweils unmittelbar nach dem Empfang entsprechender Patientendaten einen neuen aktuellen Patientendatensatz (152) basierend auf den aktuell verfügbaren Patientendaten zu bestimmen, wobei die Verarbeitungseinheit (150) weiterhin ausgebildet ist, ausgelöst durch das Bestimmen des neuen aktuellen Patientendatensatzes (152) einen Vergleich zwischen den Werten des aktuellen Patientendatensatzes (152) und den hinterlegten Zuordnungsregeln (132) zu initiieren und basierend auf diesem Vergleich mindestens eine angesichts des aktuellen Patientendatensatzes (152) empfohlene Geräteeinstellung (134) des Beatmungsgerätes (102) von der Regelspeichereinheit (130) zu empfangen, und wobei die Verarbeitungseinheit (150) ausgebildet ist, die mindestens eine empfohlene Geräteeinstellung (134) mit einer aktuellen Geräteeinstellung (154) des Beatmungsgerätes (102) zu vergleichen und basierend auf diesem Vergleich eine Empfehlungsausgabe (156) auszulösen, wobei die Empfehlungsausgabe (156) die auf der mindestens einen empfohlenen Geräteeinstellung (134) basierende Einstellungsempfehlung (105) für den mindestens einen Beatmungsparameter indiziert,
- einer Ausgabeeinheit (160), die ausgebildet ist, die Empfehlungsausgabe (156) zu empfangen und die entsprechende Einstellungsempfehlung (156) über ein Display (162) grafisch auszugeben.

2. Medizinisches System (200) gemäß Anspruch 1, wobei der Vergleich zwischen der mindestens einen empfohlenen Geräteeinstellung (134) und der aktuellen Geräteeinstellung (154) des Beatmungsgerätes nur dann zum Auslösen der Empfehlungsausgabe (156) führt, wenn ein empfohlener Stellwert für den mindestens einen Beatmungsparameters und ein aktuell vorliegender Stellwert des einzustellenden Beatmungsparameters eine vorbestimmte Stellwertmindestdifferenz (257) voneinander haben.

3. Medizinisches System (200) gemäß Anspruch 1 oder 2, wobei die Empfehlungsausgabe (156) zusätzlich die für die mindestens eine empfohlene Geräteeinstellung (134) herangezogene Zuordnungsregel (132) indiziert, und wobei die Ausgabeeinheit (260) weiter ausgebildet ist, die herangezogene Zuordnungsregel (132) über das Display (162) zumindest teilweise grafisch auszugeben.

4. Medizinisches System (200) gemäß Anspruch 2 und 3, wobei die Verarbeitungseinheit (250) ausgebildet ist, mindestens zwei empfohlene Geräteeinstellungen (134) mit der entsprechenden, herangezogenen Zuordnungsregel (132) über die Empfehlungsausgabe (156) auszugeben, falls für diese mindestens zwei empfohlenen Geräteeinstellungen (134) der entsprechend empfohlene Stellwert und der aktuell vorliegende Stellwert die jeweilige vorbestimmte Stellwertmindestdifferenz (257) voneinander haben, und wobei die Ausgabeeinheit (260) ausgebildet ist, für die mindestens zwei empfohlenen Geräteeinstellungen (134) neben der Einstellungsempfehlung (105) auch die herangezogenen Zuordnungsregeln (132) über das Display (162) zumindest teilweise grafisch auszugeben.

5. Medizinisches System (100) gemäß mindestens einem der vorhergehenden Ansprüche, wobei die Verarbeitungseinheit (150) bei Beginn einer Beatmung durch das Beatmungsgerät (102) ausgebildet ist, unmittelbar nach dem ersten empfangenen Wert eines Patientenparameters den aktuellen Patientendatensatz (152) zu bestimmen und abhängig von dem Vergleich zwischen empfohlener Geräteeinstellung (134) und aktueller Geräteeinstellung (154) die Empfehlungsausgabe (156) auszulösen.

6. Medizinisches System (100) gemäß Anspruch 5, wobei die Verarbeitungseinheit (150) bei Beginn einer Beatmung durch das Beatmungsgerät (102) ausgebildet ist, entsprechend einer vorbestimmten Bearbeitungshierarchie empfangene statische Patientendaten (112) zeitlich vor empfangenen dynamischen Patientendaten (122) für die Bestimmung des aktuellen Patientendatensatzes (152) zu berücksichtigen.

7. Medizinisches System (100) gemäß mindestens einem der vorhergehenden Ansprüche, wobei die statischen Patientendaten (112) eine Gruppe von Patientenparametern betreffen, die mindestens einen der folgenden Parameter umfasst: Patientenkategorie; Größe; Alter; Geschlecht; Gewicht; Beatmungsmodus; Vorerkrankung.

8. Medizinisches System (100) gemäß mindestens einem der vorhergehenden Ansprüche, wobei die dynamischen Patientendaten (122) eine Gruppe von Patientenparametern betreffen, die mindestens einen der folgenden Parameter umfasst: Sensordaten der Beatmung; Diagnosedaten; Blutgaswerte; Medikation; hämodynamische Kennzahl; Bettstellung; aktuelles Beatmungsmanöver; aus Sensordaten abgeleitete Daten, insbesondere Tidalvolumen und/oder Minutenvolumen.

9. Medizinisches System (100) gemäß mindestens einem der vorhergehenden Ansprüche, wobei die hinterlegten Zuordnungsregeln (132) in Form einer "Wenn-Dann"-Fallunterscheidung ausgebildet sind.

10. Medizinisches System (100) gemäß mindestens einem der vorhergehenden Ansprüche, wobei das Verarbeiten der Patientendaten durch das medizinische System (100) ausgelöst wird durch einen Empfang eines Triggersignals.

11. Medizinisches System (100) gemäß mindestens einem der vorhergehenden Ansprüche, weiterhin aufweisend eine Benutzerschnittstelle (140), die ausgebildet ist, über eine Benutzereingabe (142) eine anzuwendende Zuordnungsregel (132) zu empfangen.

12. Medizinisches System (100) gemäß Anspruch 11, wobei die Benutzerschnittstelle (140) weiterhin ausgebildet ist, eine durch einen Anwender des medizinischen Systems (100) ausgelöste Annahme einer empfohlenen Geräteeinstellung (134) zu empfangen und ein entsprechendes Annahmesignal (369) an das Beatmungsgerät (102) auszugeben.

13. Medizinisches System (100) gemäß mindestens einem der vorhergehenden Ansprüche, weiterhin aufweisend das Beatmungsgerät (102), wobei das Beatmungsgerät (102) vorliegende Beatmungsgerätedaten (104) über die Ausgabeeinheit (160) an einen Anwender des medizinischen Systems (100) ausgibt.

14. Medizinisches System (300) gemäß mindestens einem der vorhergehenden Ansprüche, wobei die Verarbeitungseinheit (350) ausgebildet ist, nach einer vorbestimmten Wartezeit, in der keine Patientendaten empfangen wurden, eine Datenanfrage (359) an ein verbundenes medizinisches Gerät auszugeben.

15. Verfahren (400) zur Bereitstellung einer Einstellungsempfehlung (105) für ein Beatmungsgerät (102) für die Beatmung eines Patienten, aufweisend die folgenden Schritte
- Hinterlegen einer Vielzahl von Zuordnungsregeln (132), wobei eine Zuordnungsregel (132) eine Zuordnung zwischen mindestens einem Wertebereich für einen Patientenparameter und einer empfohlenen Geräteeinstellung (134) für mindestens einen Beatmungsparameter des Beatmungsgerätes (102) umfasst;
- Empfangen statischer Patientendaten (112), wobei statische Patientendaten (112) Werte von Patientenparametern umfassen, die im Laufe einer Behandlungsdauer im Wesentlichen unveränderlich sind;
- Empfangen dynamischer Patientendaten (122), wobei dynamische Patientendaten (122) Werte von Patientenparametern umfassen, die sich im Laufe der Behandlungsdauer verändern;
- Bestimmen eines neuen aktuellen Patientendatensatzes (152) basierend auf den aktuell verfügbaren Patientendaten unmittelbar nach dem Empfang entsprechender Patientendaten;
- Initiieren eines Vergleichs zwischen den Werten des aktuellen Patientendatensatzes (152) und den hinterlegten Zuordnungsregeln (132), ausgelöst durch das Bestimmen des neuen aktuellen Patientendatensatzes (152);
- Empfangen einer angesichts des aktuellen Patientendatensatzes (152) empfohlenen Geräteeinstellung (134) des Beatmungsgerätes (102);
- Vergleichen der mindestens einen empfohlenen Geräteeinstellung (134) mit einer aktuellen Geräteeinstellung (154) des Beatmungsgerätes (102) und Auslösen einer Empfehlungsausgabe (156) basierend auf diesem Vergleich, wobei die Empfehlungsausgabe (156) die auf der mindestens einen empfohlenen Geräteeinstellung (134) basierende Einstellungsempfehlung (105) für den mindestens einen Beatmungsparameter indiziert;
- Empfangen der Empfehlungsausgabe (156) und grafische Ausgabe der entsprechenden Einstellungsempfehlung (105).

16. Verfahren gemäß Anspruch 15, wobei entsprechend einer vorbestimmten Bearbeitungshierarchie für die Bestimmung des aktuellen Patientendatensatzes (152) empfangene statische Patientendaten (112) zeitlich vor empfangenen dynamischen Patientendaten (122) berücksichtigt werden.
